## Europäisches Patentamt

## European Patent Office

## Office européen des brevets

(19)

(11) Veröffentlichungsnummer : **0 014 410**
**B1**

(12) **EUROPÄISCHE PATENTSCHRIFT**

(45) Veröffentlichungstag der Patentschrift :
22.09.82

(21) Anmeldenummer : 80100428.4

(22) Anmeldetag : 28.01.80

(51) Int. Cl.³ : **C 07 C 37/78**// C07C39/04,
C07C39/07

(54) Verfahren zur Abtrennung von Phenol aus einem Kresolgemisch.

(30) Priorität : 08.02.79 DE 2904831

(43) Veröffentlichungstag der Anmeldung :
20.08.80 (Patentblatt 80/17)

(45) Bekanntmachung des Hinweises auf die Patenterteilung : 22.09.82 Patentblatt 82/38

(84) Benannte Vertragsstaaten :
BE DE FR GB NL

(56) Entgegenhaltungen :
GB - A - 708 925
US - A - 3 221 063
US - A - 3 331 755
US - A - 3 337 424
US - A - 3 397 124
US - A - 3 392 090
US - A - 3 483 094
CHEMICAL ABSTRACTS, Band 78, Nr. 21, 28.
Mai 1973, Seite 345, Nr. 135859 t Columbus, Ohio,
U.S.A.
CHEMICAL ABSTRACTS, Band 87, Nr. 3, 18. Juli
1977, Seite 596, Nr. 22649 s Columbus, Ohio,
U.S.A.
G. A. KIRICHENKO et al. : « Thermodynamictopological analysis of a system for separation of
products in 2,6-xylenol production »

(73) Patentinhaber : BAYER AG
Zentralbereich Patente, Marken und Lizenzen
D-5090 Leverkusen 1, Bayerwerk (DE)

(72) Erfinder : Wüst, Alfredo, Dr.
Carl-Rumpff-Strasse 55
D-5090 Leverkusen 1 (DE)
Erfinder : Hammerström, Knut, Dr.
Pehlengarten 3
D-5060 Bergisch-Gladbach 1 (DE)

**0 014 410**

Verfahren zur Abtrennung von Phenol aus einem Kresolgemisch

Die Erfindung betrifft ein Verfahren zur Abtrennung von Phenol aus Kresolgemischen.

Eine Abtrennung von Phenol aus einem Gemisch isomerer Kresole ist aufwendig, da die Siedepunkte nahe zusammenliegen und somit eine destillative Trennung erschwert ist. Durch die chemische Ähnlichkeit der Komponenten bedingt, sind andere Trennverfahren, beispielsweise chemische Trennverfahren, sehr schwierig und kommen für die technische Nutzung kaum in Frage.

Besonders schwierig ist die Abtrennung des Phenols, wenn kleine Phenolgehalte im Kresol, beispielsweise unter 2 Gew.-%, abgetrennt werden sollen und wenn das Kresol nur noch sehr wenig Phenol, beispielsweise unter 0,5 Gew.-%, enthalten soll. Kleine Mengen an Phenolen können beispielsweise bei der Herstellung von Kresolen durch Hochtemperaturverseifung von Chlortoluolen mit Natronlauge (US 1 996 744) als Nebenprodukt anfallen.

Aus Chemical Abstract *87* (1977), S. 596, 22649s ist bekannt, 2,6-Xylenol durch Destillation von anderen bei der Produktion des 2,6-Xylenol anfallenden Nebenprodukten, wie Kresolen, Anisol und nicht reagiertem Phenol und Methanol durch Destillation abzutrennen, indem man zunächst das Methanol abdestilliert, dann dem Gemisch Toluol zusetzt und ein Wasser/Toluolazeotrop-Gemisch überdestilliert und anschließend nacheinander ein Phenol/Anisol-Gemisch, o-Kresol und ein Gemisch bestehend aus m- und p-Kresol abdestilliert. Eine Abtrennung des Phenols von den Kresol-Isomeren durch Zusatz eines Azeotropbildners findet dabei nicht statt.

Auch aus der US-PS 3 483 094 ist nicht zu entnehmen, daß man Phenol aus Gemischen, die im wesentlichen aus Phenol und Kresol bestehen, durch Zusatz einer Substanz, die mit Phenol ein Azeotrop zu bilden vermag, abtrennen kann. Aus der Tabelle II der US-PS ist nur zu entnehmen, daß man 2,6-Xylenol von o-, m- und p-Kresol sowie von Phenol durch Destillation abtrennen kann, wenn man dem zu destillierenden Gemisch Alkylene mit 10 bis 13 Kohlenstoffatomen, wie Dodecen-1, zusetzt. Eine Trennung des Phenols von den kresolen ist aus der Tabelle II nicht ersichtlich, vielmehr sind Phenol und die Kresole in den zuerst übergehenden Fraktionen stets gleichzeitig vorhanden, wobei kaum eine merkliche Anreicherung des Phenols in den Fraktionen zu beobachten ist.

Es wurde nun ein Verfahren zur Abtrennung von Phenol aus Gemischen, die im wesentlichen aus Phenol und Kresol bestehen, gefunden, das dadurch gekennzeichnet ist, daß man das Gemisch in Gegenwart von gegebenenfalls halogenierten, gesättigten oder ungesättigten aliphatischen Kohlenwasserstoffen mit einem Dipolmoment von 0 bis 0,5 Debye und einem Siedepunkt bei 1 bar von 120 bis 220 °C und/oder in Gegenwart von cycloaliphatischen Kohlenwasserstoffen mit einem Dipolmoment von 0 bis 2 Debye und einem Siedepunkt bei 1 bar von 150 bis 190 °C und/oder in Gegenwart von gegebenenfalls halogenierten Alkylbenzolen mit einem Dipolmoment von 0 bis 2,5 Debye und einem Siedepunkt bei 1 bar von 150 bis 190 °C und/oder in Gegenwart von alkylierten Halogenphenolen mit einem Dipolmoment von 0 bis 2,5 Debye und einem Siedepunkt bei 1 bar von 150 bis 190 °C destilliert.

Kresol für das erfindungsgemäße Verfahren kann ortho-, meta- oder para-Kresol oder auch ein Gemisch der verschiedenen isomeren Kresole sein.

Die Komponenten, die für die Destillation nach dem erfindungsgemäßen Verfahren zugesetzt werden können, bilden im allgemeinen mit dem Phenol oder mit dem Phenol und dem Kresol, bevorzugt mit dem Phenol, ein Azeotrop.

Als Zusatzkomponenten für die Destillation nach dem erfindungsgemäßen Verfahren seien beispielsweise genannt: gegebenenfalls halogenierte, gesättigte oder ungesättigte aliphatische Kohlenwasserstoffe, cycloaliphatische Kohlenwasserstoffe, gegebenenfalls halogeniertes Alkylbenzol und gegebenenfalls alkyliertes Halogenphenol.

Gegebenenfalls halogenierte, gesättigte oder ungesättigte aliphatische Kohlenwasserstoffe für das erfindungsgemäße Verfahren haben ein Dipolmoment von 0 bis 0,5 Debye, bevorzugt von 0 bis 0,2 Debye, und einen Siedepunkt bei 1 bar von 120 bis 220 °C, bevorzugt von 150 bis 190 °C. Im allgemeinen handelt es sich um geradkettige oder verzweigte aliphatische Kohlenwasserstoffe mit etwa 2 bis etwa 12 Kohlenstoffatomen. Die Kohlenwasserstoffe können eine oder mehrere Doppelbindungen haben und durch ein oder mehrere Halogenatome, wie Fluor, Chlor, Brom oder Jod, bevorzugt Chlor, substituiert sein.

Als gegebenenfalls halogenierte, gesättigte oder ungesättigte Kohlenwasserstoffe seien beispielsweise genannt: Pentachloräthan, Hexachloräthan, Octan, Decan, Dodecan, 1-Decen.

Bevorzugte gegebenenfalls halogenierte, gesättigte oder ungesättigte Kohlenwasserstoffe für das erfindungsgemäße Verfahren sind Pentachloräthan, n-Decan.

Cycloaliphatische Kohlenwasserstoffe für das erfindungsgemäße Verfahren haben ein Dipolmoment von 0 bis 2 Debye, bevorzugt von 0 bis 1,75 Debye und einen Siedepunkt bei 1 bar von 150 bis 200 °C, bevorzugt von 150 bis 190 °C. Im allgemeinen handelt es sich um Terpene.

Als cycloaliphatische Kohlenwasserstoffe für das erfindungsgemäße Verfahren seien beispielsweise genannt: α-Limonen, Camphen, α-Pinen.

Bevorzugte cycloaliphatische Kohlenwasserstoffe für das erfindungsgemäße Verfahren sind Camphen, α-Pinen.

2

Gegebenenfalls halogeniertes Alkylbenzol für das erfindungsgemäße Verfahren hat ein Dipolmoment von 0 bis 2,5 Debye, bevorzugt von 0 bis 2,0 Debye, und einen Siedepunkt bei 1 bar von 150 bis 190 °C. Im allgemeinen handelt es sich um durch niedere (1 bis etwa 6 Kohlenstoffatome) geradkettige oder verzweigte Alkylreste substituiertes Benzol, das durch ein oder mehrere Halogenatome, wie Fluor, Chlor, Brom oder Jod, bevorzugt chlor, im aromatischen oder aliphatischen Teil des Moleküls substituiert sein kann.

Als gegebenenfalls halogenierte Alkylbenzole für das erfindungsgemäße Verfahren seien beispielsweise genannt : p-Dichlorbenzol, o- und p-Chlortoluol, o-, m- und p-Bromtoluol, Isopropylbenzol, Mesitylen. sec-Butylbenzol, Methyläthylbenzol.

Bevorzugte gegebenenfalls halogenierte Alkylbenzole für das erfindungsgemäße Verfahren sind o- und p-Chlortoluol, Mesitylen, Methyläthylbenzol.

Gegebenenfalls alkyliertes Halogenphenol für das erfindungsgemäße Verfahren besitzt ein Dipolmoment von 0 bis 2,5 Debye, bevorzugt von 0 bis 2 Debye, und einen Siedepunkt bei 1 bar von 150 bis 190 °C, bevorzugt von 155 bis 180 °C. Im allgemeinen handelt es sich um durch Fluor, Chlor, Brom oder Jod, bevorzugt Chlor, substituiertes Phenol, das durch einen niederen (1 bis 6 Kohlenstoffatome) geradkettigen oder verzweigten Alkylrest substituiert sein kann.

Als gegebenenfalls alkylierte Halogenphenole für das erfindungsgemäße Verfahren seien beispielsweise genannt : o-Chlorphenol, o-Bromphenol.

Bevorzugtes gegebenenfalls alkyliertes Halogenphenol für das erfindungsgemäße Verfahren ist o-Chlorphenol.

Es ist selbstverständlich möglich, ein Gemisch der bei der Destillation erfindungsgemäß zugesetzten Komponenten einzusetzen.

Die Trennung nach dem erfindungsgemäßen Verfahren wird in Form einer Azeotropdestillation durchgeführt.

Die Trennung nach dem erfindungsgemäßen Verfahren kann diskontinuierlich oder kontinuierlich durchgeführt werden. Es ist möglich, die zugesetzte Komponente nach dem Trennverfahren abzutrennen und erneut in die Trennung einzusetzen.

Das erfindungsgemäße Verfahren kann beispielsweise wie folgt durchgeführt werden :

Dem Phenol enthaltenden Kresol wird vor der Destillation die Zusatzkomponente zugegeben. Dies kann beispielsweise dadurch geschehen, daß man das Phenol enthaltende Kresol und die Zusatzkomponente getrennt in die Destillationskolonne einleitet. Im allgemeinen gibt man 0,01 bis 0,4 Teile, bevorzugt 0,02 bis 0,1 Teile, der Zusatzkomponente zu einem Teil des phenol enthaltenden Kresols.

Die Destillation wird im allgemeinen im Temperaturbereich von 120 bis 220 °C, bevorzugt von 150 bis 210 °C, und unter Normaldruck durchgeführt. Falls die Destillation bei einem anderen Druck durchgeführt wird, ändern sich die Temperaturbereiche selbstverständlich entsprechend.

Bei der Destillation wird das Kresol von dem Phenol und der Zusatzkomponente getrennt. Aus dem Sumpf der Kolonne kann das phenolfreie Kresol entnommen werden. Über Kopf der Kolonne erhält man ein Gemisch aus Phenol und der Zusatzkomponente, das praktisch kein Kresol enthält.

Das über Kopf der Destillationskolonne erhaltene Gemisch kann in einer zweiten Destillationskolonne mit Hilfe eines Schleppmittels für die bei der erfindungsgemäßen Trennung eingesetzten Zusatzkomponente in seine Bestandteile zerlegt werden. Als Schleppmittel seien beispielsweise Wasser, n-Decan, bevorzugt Wasser, genannt.

Bei der Destillation des Phenols, der Zusatzkomponente und des Wassers erhält man im Sumpf der Destillationskolonne reines Phenol und über Kopf ein Gemisch aus Wasser, der Zusatzkomponente und gegebenenfalls Spuren von Phenol. Nach Kondensation des über Kopf der Destillationskolonne erhaltenen Gemisches, erhält man ein Gemisch das aus einer wäßrigen und einer organischen Phase besteht. Insbesondere bei kontinuierlicher Arbeitsweise führt man die wäßrige Phase in die zweite Kolonne zurück und leitet die organische Phase, die im wesentlichen aus der Zusatzkomponente besteht, in die erste Kolonne zur erfindungsgemäßen Trennung von phenolhaltigem Kresol zurück.

Nach dem erfindungsgemäßen Verfahren kann in einfacher Weise Phenol von Kresol getrennt werden. Es ist leicht möglich, kleinere Mengen an Phenol und Spuren von Phenol von Kresol abzutrennen.

Bei der chemischen Ähnlichkeit von Phenol und Kresol war es nicht zu erwarten, Zusatzkomponenten zu finden, durch die eine destillative Trennung von Phenol und Kresol möglich ist.

Beispiele

I. Diskontinuierliche Abtrennung von Phenol aus einem Kresolgemisch

Beispiel 1

3 969 g eines Kresolisomeren-Gemisches der analytischen Zusammensetzung

Wasser weniger als 0,01 %
Phenol 0,24 %

o-Kresol 25,65 %
m-Kresol 50,52 %
p-Kresol 23,59 %

werden mit 435 g o-Chlortoluol versetzt. Die Destillation dieser Mischung erfolgt bei einem Vakuum von 400 mbar und einem Rücklaufverhältnis von 1:10 an einer 1,2 m langen Füllkörperkolonne (evakuiert und verspiegelt ; 5 × 5 mm — Drahtnetz-Raschigringe).

Die bei 119 °C Kopftemperatur übergehende Fraktion (423 g) enthält 1,6 % Phenol und ist frei von o-Kresol. Danach beginnt die Kopftemperatur zu steigen und es wird noch eine weitere Fraktion bis zu 144 °C im Kopf abgenommen. Diese Nachfraktion (25 g) besteht zu 18 % aus o-Chlortoluol, 4,5 % Phenol und 77,5 % o-Kresol.

Der Sumpf (3 914 g) ist frei von o-Chlortoluol und Phenol.

## Beispiel 2

Zu 3 747 g eines entwässerten Kresolisomeren-Gemisches (Zusammensetzung wie in Beispiel 1) mit einem Phenolgehalt von 0,26 % werden 428 g p-Chlortoluol gegeben.

Die Destillation wird gemäß der in Beispiel 1 gegebenen Vorschrift durchgeführt.

Die bei 128 °C Kopftemperatur übergehende Fraktion (418 g) enthält 2,0 % Phenol und ist frei von o-Kresol. Die Nachfraktion (27 g) wird bis zu einer Kopftemperatur von 154 °C abgenommen und setzt sich aus 23 % p-Chlortoluol, 4,8 % Phenol und 72,2 % o-Kresol zusammen.

Im Sumpf (3 698 g) können weder p-Chlortoluol noch Phenol nachgewiesen werden.

## Beispiel 3

Zu 4 163 g eines entwässerten Rohkresol-Gemisches (Zusammensetzung wie in Beispiel 1) mit einem Phenolgehalt von 0,32 % werden 432 g eines Chlortoluolisomeren-Gemisches (53 % o-Chlortoluol, 47 % p-Chlortoluol) gegeben und gemäß der im Beispiel 1 gegebenen Destillationsvorschrift verfahren.

Die bei 124 bis 126 °C Kopftemperatur übergehende Fraktion (422 g) ist o-kresolfrei und enthält 2,1 % Phenol. Die Nachfraktion (24 g) wird bis zu einer Kopftemperatur von 151 °C abgenommen und besteht zu 22 % aus Chlortoluol isomeren, 6,3 % Phenol und 71,7 % o-Kresol.

Der Sumpf (4 102 g) ist frei von Chlortoluolisomeren und Phenol.

II. Kontinuierliche Abtrennung von Phenol in einem Roh-Kresolgemisch

## Beispiel 4

Eine Kresolmischung der Zusammensetzung

Wasser weniger als 0,01 %
Phenol 0,24 %
o-Kresol 26,09 %
m-Kresol 50,71 %
p-kresol 22,96 %

wird durch Zusetzen von Phenol und einem Chlortoluolisomerengemisch (53 % o-Chlortoluol, 47 % p-Chlortoluol) auf einen Phenolgehalt von 0,5 % und einem Chlortoluolisomeren-Gehalt von 3,5 % eingestellt.

Die Aufarbeitung dieser Mischung erfolgt in einer Destillierapparatur gemäß dem folgenden Fließbild.

## Beschreibung des Fließbilds

Die verwendete Destillierapparatur besteht aus dem Kolonnensumpf 1, dem Abtriebsteil 2, dem Verstärkeiteil 3, dem Kolonnenkopf 4 und den Dosierpumpen P1 und P2.

Verstärker- und Abtriebsteil sind übliche Füllkörperkolonnen (evakuiert und verspiegelt ; 4 × 4 mm — Drahtnetz-Raschigringe).

Das Phenol enthaltende Kresol und das chlortoluol werden entsprechend dem Fließbild in einem Mengenstrom von 200 g/h zwischen Verstärker- und Abtriebsteil eingeführt.

Die Anlage wird bei Normaldruck mit einem Rücklaufverhältnis von 1 : 50 betrieben.

Am Kopf der Apparatur werden stündlich bei 156 °C etwa 8 g eines o-kresolfreien Phenol-Chlortoluolisomeren-Gemisches abgenommen. Aus dem Kolonnensumpf, der bei 201 °C gehalten wird, können in ebenfalls kontinuierlicher Arbeitsweise etwa 190 g Kresol pro Stunde entnommen werden.

Bei dieser Arbeitsweise ist der Sumpf frei von Phenol und Chlortoluolisomeren.

## Beispiele 5 bis 7

In der folgenden Tabelle sind die Ergebnisse von drei Beispielen zusammengefaßt, die analog Beispiel 4 durchgeführt werden. Dabei wird in allen Fällen eine Kresolmischung in der Zusammensetzung wie in Beispiel 4 eingesetzt.

Anstelle von Chlortoluolisomeren wird Mesitylen (Trimethylbenzol), Camphen und p-Dichlorbenzol als Schleppmittel für Phenol verwendet. Der Phenolgehalt beträgt jeweils 0,5 %.

|  | Mesitylen | Camphen | p-Dichlorbenzol |
|---|---|---|---|
| Einlaufmenge g/h | 250 | 150 | 200 |
| Schleppmittelkonzentration in % | 2,5 | 3,0 | 3,0 |
| Kopfabnahme g/h | 7 | 5 | 7 |
| Sumpfaustrag g/h | 240 | 145 | 190 |
| Kopftemperatur °C | 163 | 147 | 135 |
| Sumpftemperatur °C | 200 | 202 | 205 |

III. Diskontinuierliches Zerlegen von Phenol-Schleppmittel-Gemischen mit Wasser

### Beispiel 8

Ein Gemisch aus 100 g Phenol, 400 g o-Chlortoluol und 600 g Wasser wird an einer Füllkörperkolonne (technische Ausstattung wie in Beispiel 1) einer fraktionierten Destillation bei Normaldruck unterworfen. Bei einer Kopftemperatur von 96 °C und einem Rücklaufverhältnis von 1 : 10 werden zunächst 926 g eines Zweiphasengemisches aus Wasser und o-Chlortoluol abgenommen. Die Wasserphase ist frei von Phenol, während in der o-Chlortoluol-Phase weniger als 0,04 % Phenol nachgewiesen werden können.

Anschließend wird noch ein Zwischenlauf abdestilliert. beim Erreichen einer Kopftemperatur von 181 °C wird die Destillation abgebrochen.

Während sich der Zwischenlauf (57 g) aus Wasser, o-Chlortoluol und Phenol zusammensetzt, besteht der Kolonnensumpf (64 g) aus reinem Phenol.

### Beispiel 9

Ein Gemisch aus 100 g Phenol, 700 g Wasser und 700 g eines Isomerengemisches aus 53 % o-Chlortoluol und 47 % p-Chlortoluol wird analog den im Beispiel 8 genannten Bedingungen destilliert.

Bei 96 °C im Kopf werden 1 312 g eines Zweiphasengemisches aus Wasser und den beiden Chlortoluol-Isomeren abgenommen. Beide Phasen sind frei von Phenol.

Der Zwischenlauf (insgesamt 61 g) wird ebenfalls bis zu einer Kopftemperatur von 181 °C abdestilliert und enthält die vier Einsatzkomponenten.

Der Kolonnensumpf (55 g) besteht aus Phenol und ist frei von Wasser und den Chlortoluol-Isomeren.

IV. Kontinuierliches Zerlegen von Phenol-Schleppmittel-Gemischen mit Wasser

Eine hierfür geeignete Apparatur ist ähnlich wie die im Beispiel 4 beschriebene Anlage aufgebaut. Es wird lediglich der Kolonnenkopf 4 durch einen Kühler mit integriertem Phasenabscheider ersetzt. Aus dem Abscheider kann wahlweise die obere bzw. Untere Phase als Rücklauf auf die Kolonne gegeben werden, während die jeweils verbleibende Phase in den Ablauf geht. Es wird bei Normaldruck gearbeitet.

### Beispiel 10

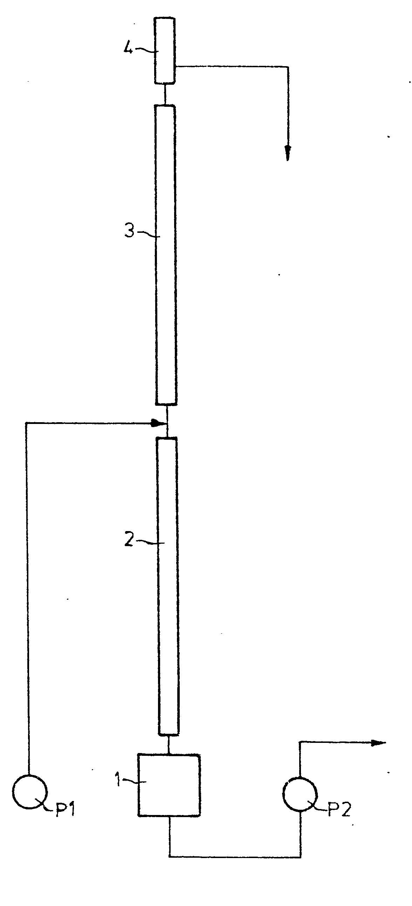

Im Destillationskolben 1 gemäß der Abbildung wird ein Gemisch aus 500 g Phenol und 100 g Wasser vorgelegt. Nach dem Anheizen wird mit der Pumpe P1 eine Lösung von 800 g Phenol in 3 200 g Chlortoluolisomerengemisch (53 % o-Chlortoluol, 47 % p-Chlortoluol) in einer Rate von 150 ml/h zwischen Abtriebs- und Verstärkerteil der Anlage eingeführt. Das anfallende Kopfdestillat trennt sich im Abscheider in eine obere Wasser- und eine untere Chlortoluolphase auf. Die Wasserphase geht voll als Rücklauf auf die Kolonne, während die Chlortoluolphase kontinuierlich abgenommen wird. Gleichzeitig wird unter Konstanthaltung des Sumpfniveaus kontinuierlich Sumpfprodukt abgepumpt.

Nachdem sich Sumpftemperatur auf 182 °C und Kopftemperatur auf 96 °C eingestellt haben, können bei einer Einlaufmenge von 150 ml/h etwa 120 ml Chlortoluolisomere pro Stunde am Abscheider abgenommen werden. Bei dieser Fahrweise ist der Sumpfaustrag (im Mittel 30 ml/h) frei von Wasser und Chlortoluol-Isomeren, während der Phenolgehalt der abgehenden Chlortoluolisomeren bei 1 bis 2 % liegt.

0 014 410

## Beispiel 11

Es wird die gleiche Apparatur wie in Beispiel 10 verwendet.

Im Destillationskolben wird ein Gemisch aus 500 g Phenol und 100 g Wasser vorgelegt und nach dem Anheizen eine Lösung aus 800 g Phenol in 3 200 g Mesitylen wie beschrieben in die Apparatur eingeführt.

Das Kopfkondensat trennt sich im Abscheider in eine obere Mesitylen- und in eine untere Wasserphase auf. Das Mesitylen wird kontinuierlich abgenommen, während das Wasser als Rücklauf auf die Kolonne zurückgeführt wird.

Nach Ermittlung aller Parameter (182 °C Sumpftemperatur, 95 °C Kopftemperatur, 190 ml/h Einlauf, 160 ml/h Kopfabnahme und 30 ml/h Sumpfentnahme) ist der Sumpf frei von Wasser und Mesitylen. Das am Abscheider anfallende Mesitylen enthält 2 bis 3 % Phenol.

## Beispiel 12

Es wird die in Beispiel 10 beschriebene Apparatur verwendet.

Im Destillationskolben wird ein Gemisch aus 100 g n-Decan und 500 g entwässerten Rohkresolisomeren vorgelegt. Die Zusammensetzung des Rohkresols ist ähnlich wie im Beispiel 1 beschrieben.

Nach dem Anheizen werden pro Stunde kontinuierlich 200 ml Rohkresole obiger Zusammensetzung in die Anlage zwischen Abtriebs- und Verstärkerteil eindosiert. Es wird bei Normaldruck gearbeitet.

Die Sumpftemperatur wird auf 205 °C gebracht, dabei stellt sich eine Kopftemperatur von 157 °C ein.

Im Abscheider fällt ein Zweiphasengemisch an, mit n-Decan als obere und Phenol als untere Phase. Jede der beiden Phasen enthält etwa 10 % der anderen Komponenten.

Die n-Decan-Phase wird als Rücklauf auf die Kolonne zurückgeführt, während die Phenolphase von Zeit zu Zeit abgelassen wird.

Bei einem Einlauf von 200 ml/h hönnen am Abscheider im Mittel 0,5 ml/h Phenolphase abgenommen werden. Der Sumpfaustrag (ca. 200 ml/h) ist phenolfrei.

## Beispiel 13

Apparativ und verfahrensmäßig wird wie im Beispiel 12 beschrieben verfahren.

Anstelle von n-Decan wird ein Kohlenwasserstoff-Schnitt (170 bis 175 °C bei Normaldruck) verwendet, der durch Fraktionieren von Testbenzin an einer 1,2 m langen Füllkörperkolonne bei einem Rücklaufverhältnis von 1 : 10 erhalten wird.

Im Abscheider fällt eine obere Kohlenwasserstoff und eine untere Phenolphase an. Die Kohlenwasserstoff-Phase wird als Rücklauf zurückgeführt, die Phenolphase wird abgenommen.

Nach Einstellung konstanter Versuchsbedingungen (Temperaturen und Mengenströme entsprechen den im Beispiel 12 angegebenen Daten) ist der Sumpfaustrag frei von Phenol und Kohlenwasserstoffen. Die Phenolphase aus dem Abscheider enthält durchschnittlich 51 % Kohlenwasserstoffe.

## Beispiel 14

Ein Gemisch aus 900 g Phenol und 100 g n-Decan wird in einem Destillierkolben vorgelegt. Die Destillation erfolgt anschließend bei Normaldruck über eine 1,2 m lange Füllkörperkolonne (wie in Beispiel 1 beschrieben), die mit einem Kühler mit integriertem Phasenabschneider versehen ist.

Die sich abscheidende Phenolphase wird als Rücklauf auf die Kolonne zurückgeführt, die n-Decan-Phase wird abgenommen. Sobald im Abscheider keine Phasenabtrennung mehr erfolgt, wird die Destillation abgebrochen. Der Sumpf ist dann frei von n-Decan.

Der Phenolgehalt der abgenommenen n-Decan-Phase beträgt im Mittel 10,5 %.

**Anspruch**

Verfahren zur Abtrennung von Phenol aus Gemischen, die im wesentlichen aus Phenol und Kresol bestehen, dadurch gekennzeichnet, daß mann das Gemisch in Gegenwart von gegebenenfalls halogenierten, gesättigten oder ungesättigten aliphatischen Kohlenwasserstoffen mit einem Dipolmoment von 0 bis 0,5 Debye und einem Siedepunkt bei 1 bar von 120 bis 220 °C und/oder in Gegenwart von cycloaliphatischen Kohlenwasserstoffen mit einem Dipolmoment von 0 bis 2 Debye und einem Siedepunkt bei 1 bar von 150 bis 190 °C und/oder in Gegenwart von gegebenenfalls halogenierten Alkylbenzolen mit einem Dipolmoment von 0 bis 2,5 Debye und einem Siedepunkt bei 1 bar von 150 bis 190 °C und/oder in Gegenwart von alkylierten Halogenphenolen mit einem Dipolmoment von 0 bis 2,5 Debye und einem Siedepunkt bei 1 bar von 150 bis 190 °C destilliert.

**Claim**

Process for separating off phenol from mixtures which consist essentially of phenol and cresol, characterised in that the mixture is distilled in the presence of optionally halogenated, saturated or unsaturated aliphatic hydrocarbons with a dipole moment of 0 to 0.5 Debyes and a boiling point, under 1 bar, of 120 to 220 °C and/or in the presence of cycloaliphatic hydrocarbons with a dipole moment of 0 to 2 Debyes and a boiling point, under 1 bar, of 150 to 190 °C and/or in the presence of optionally halogenated alkyl benzenes with a dipole moment of 0 to 2.5 Debyes and a boiling point, under 1 bar, of 150 to 190 °C and/or in the presence of alkylated halogenophenols with a dipole moment of 0 to 2.5 Debyes and a boiling point, under 1 bar, of 150 to 190 °C.

**Revendication**

Procédé de séparation de phénol à partir de mélanges qui consistent essentiellement en phénol et crésol, caractérisé en ce qu'on distille le mélange en présence d'hydrocarbures aliphatiques saturés ou non saturés éventuellement halogénés ayant un moment dipôle de 0 à 0,5 Debye et un point d'ébullition sous 1 bar de 120 à 220 °C et/ou en présence d'hydrocarbures cycloaliphatiques ayant un moment dipôle de 0 à 2 Debye et un point d'ébullition sous 1 bar de 150 à 190 °C et/ou en présence d'alcoylbenzènes éventuellement halogénés ayant un moment dipôle de 0 à 2,5 Debye et un point d'ébullition sous 1 bar de 150 à 190 °C et/ou en présence d'halophénols alcoylés ayant un moment dipôle de 0 à 2,5 Debye et un point d'ébullition sous 1 bar de 150 à 190 °C.

7